# EUROPEAN PATENT APPLICATION

(11) **EP 2 308 899 A1**
(43) Date of publication of application: **13.04.2011**
(21) Application number: 09773469.3
(22) Date of filing: 30.06.2009
(51) Int. Cl.: C07K 16/46, C12N 15/09, C12Q 1/68, G01N 33/532

(54) **ANTIBODY COMPLEX, ANTIGEN DETECTION METHOD, AND METHOD FOR PRODUCTION OF ANTIBODY COMPLEX**

(30) Priority: 30.06.2008 JP 2008171512
(71) Applicant: Tokyo Metropolitan Organization for Medical Research, Tokyo 156-0057 (JP); Synthera Technologies Co., Ltd., Bunkyo-ku Tokyo 113-0021 (JP)
(72) Inventor: SHIBASAKI, Futoshi, Tokyo 175-0094 (JP); MORIZANE, Yoshihito, Tokyo 113-0021 (JP)
(74) Representative: Denison, Christopher Marcus
(86) International application number: PCT/JP2009/061959
(87) International publication number: WO 2010/001891

(57) **Abstract**

In order to improve the detection sensitivity of MUSTag, the present invention provides an antibody complex including a nucleic acid chain as a label, an antibody to specifically recognize the antigen and an adaptor moiety linking the nucleic acid chain and the antibody, wherein the adaptor moiety includes an immunoglobulin binding domain of Protein G, Protein A or Protein L for binding with the antibody, and the adaptor moiety and the antibody are chemically cross-linked to form a cross-linked antibody complex.

## Description

### [Cross-Reference to Related Applications]

The present application claims the priority based on Japanese Patent Application No.2008-171512 filed on June 30, 2008, and the disclosure of this parent application is herein incorporated by reference.

### [Technical field]

The present invention relates to antibody complexes, methods for detecting an antigen using the antibody complex and methods for producing the antibody complex.

### [Background Art]

For detecting a small amount of antigen, methods such as ELISA have been developed. Recently, a method which utilizes a complex called MUSTag having improved detection sensitivity was developed (International Patent Publication WO2006/049289). In a specific example of this method, using a complex made by linking an oligonucleotide as a label to an antibody via Protein G, an antigen is detected by allowing the antibody moiety of this complex to bind to the antigen, then cleaving and recovering the oligonucleotide and detecting the oligonucleotide by PCR.

### [Summary of Invention]

### [Technical Problem]

An object of the present invention is to improve detection sensitivity of MUSTag.

### [Solution to Problem]

In order to improve the sensitivity of detection of MUSTag, the Applicant has explored various conditions and discovered that, as disclosed in the Examples herein, the sensitivity of detection of an antigen could be intensified by cross-linking an adaptor moiety and the antibody. As it has been known that Protein G, Protein A and Protein L bind to the Fc region of an IgG molecule with high affinity, this discovery was unexpected for those skilled in the art, suggesting that in the methods for detecting an antigen by using MUSTag, the binding capability of Protein G, Protein A or Protein L to the antibody was not sufficient.

In one embodiment of the present invention, an antibody complex for detecting an antigen includes a nucleic acid chain as a label, an antibody to specifically recognize the antigen, and an adaptor moiety linking the nucleic acid chain and the antibody, wherein the adaptor moiety includes an immunoglobulin binding domain of Protein G, Protein A or Protein L, and the adaptor moiety and the antibody are chemically cross-linked. The antibody complex may include a cleavage site at which the nucleic acid chain can be released. The cleavage site may be cleaved by a restriction enzyme, by photoirradiation or by reactive oxygen. The adaptor moiety may include a fusion protein including a biotin binding domain of an avidin and the immunoglobulin binding domain of Protein G, Protein A or Protein L. The nucleic acid chain may be a biotin-conjugated nucleic acid. The antibody may be a monoclonal antibody.

In another embodiment of the present invention, a method for detecting an antigen includes a step of allowing the antibody complex to contact with the antigen, thereby forming an antigen-antibody complex including the antigen and the antibody complex, and a detection step of detecting the oligonucleotide chain. The antigen-antibody complex may include the antibody complex, and the detection step may include a step of releasing the oligonucleotide chain from the cleavage site and recovering the oligonucleotide chain. The detection step may also include a step of amplifying the oligonucleotide chain and a step of detecting the amplified oligonucleotide.

In yet another embodiment of the present invention, a kit for detecting an antigen includes a nucleic acid chain as a label, an antibody to specifically recognize the antigen, and an antibody complex including an adaptor moiety linking the nucleic acid chain and the antibody, wherein the adaptor moiety includes an immunoglobulin binding domain of Protein G, Protein A or Protein L, and the adaptor moiety and the antibody are chemically cross-linked. The antibody complex may include a cleavage site capable of releasing the nucleic acid chain. The cleavage site may be cleaved by a restriction enzyme, by photoirradiation or by reactive oxygen. The adaptor moiety may include a fusion protein including a biotin-binding domain of an avidin and the immunoglobulin binding domain of Protein G, Protein A or Protein L, and the nucleic acid chain may be a biotin-conjugated nucleic acid. The antibody may be a monoclonal antibody.

### [Brief Description of Drawings]

[Fig.1]

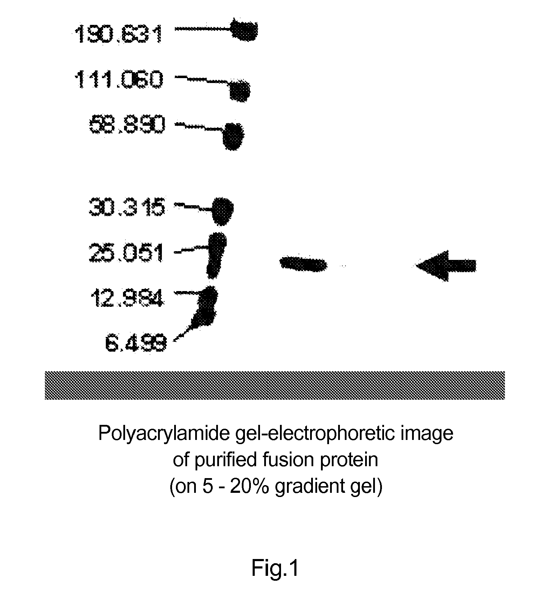

   Fig.1 is a photograph showing a result of SDS-PAGE using the purified fusion protein of Protein G / streptavidin / His-tag in one embodiment of the present invention.
[Fig.2]

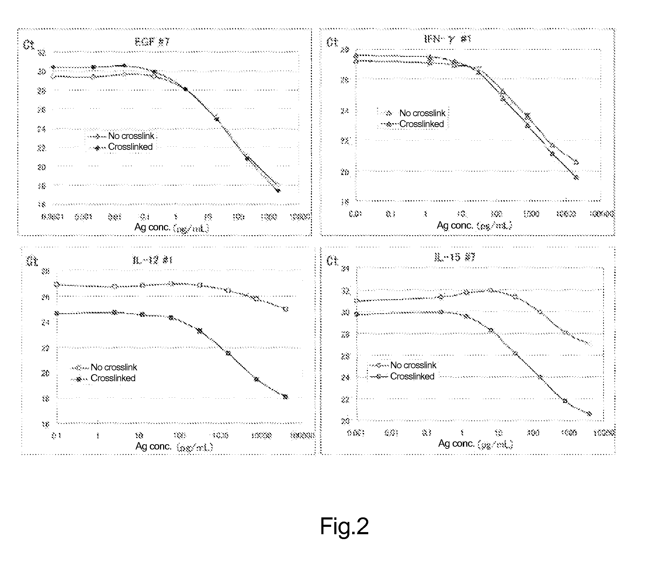

   Fig.2 is a set of graphs showing results of the antigen detection at various antigen concentrations using a cross-linked MUSTag in one embodiment of the present invention.
[Fig.3]

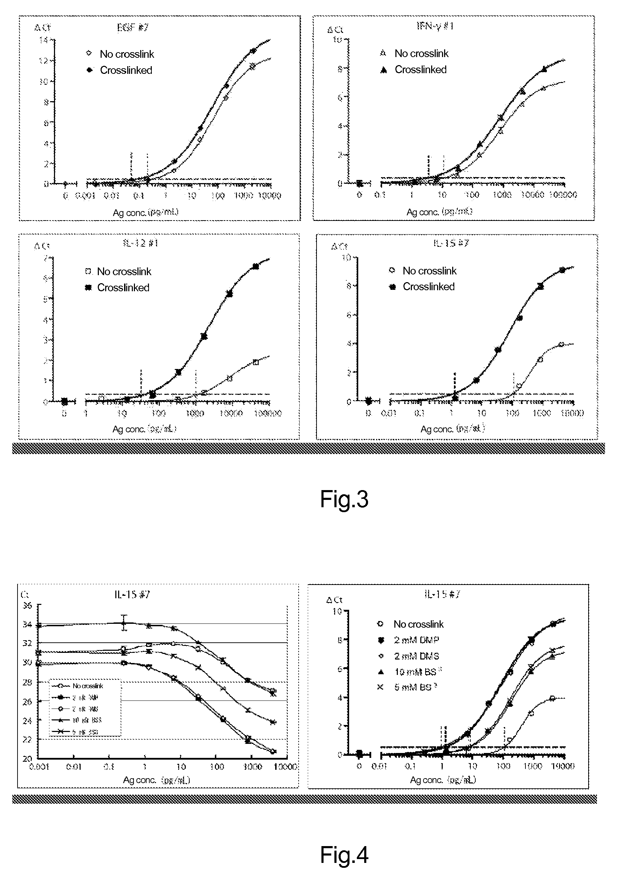

   Fig.3 is a set of graphs showing normalized results of the antigen detection at various antigen concentrations using a cross-linked MUSTag in one embodiment of the present invention.
[Fig.4]
   Fig.4 is graphs showing the results or normalized results of the antigen detection at various antigen concentrations using cross-linked MUSTags produced with several cross-linkers in one embodiment of the present invention.

### [Description of Embodiments]

Embodiments of the present invention accomplished based on the abovementioned discovery are hereinafter described in detail by referring to Examples. Unless otherwise explained in the Description of Embodiments or Examples, methods described in standard sets of protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., as well as their modifications/variations are employed. When a commercially available reagent kit or a measuring instrument is used, the protocol attached thereto will be followed unless otherwise explained.

The object, characteristics, and advantages of the present invention as well as the idea thereof will be apparent to those skilled in the art from the descriptions given herein, and the present invention can be easily reproduced by those skilled in the art based on the descriptions given herein. The embodiments and specific examples of the invention described herein are to be taken as preferred embodiments of the present invention, and these descriptions are presented only for illustrative and/or explanatory purposes and should not be construed as limiting the present invention thereto. It is apparent to those skilled in the art that various changes and modifications may be made based on the descriptions given herein within the intent and scope of the present invention disclosed herein.

### == Cross-linked antibody complex ==

The antibody complex according to the present invention includes a nucleic acid chain as a label, an antibody which specifically recognizes an antigen to be detected, and an adaptor moiety which links the nucleic acid chain and the antibody, wherein the adaptor moiety includes an immunoglobulin binding domain of Protein G, Protein A or Protein L, and the adaptor moiety and the antibody are chemically cross-linked.

The nucleic acid chain as a label may be a DNA or an RNA, but it is preferably a DNA because of its easier detection. Its length is not particularly limited, but shorter chains are more preferable so that an enzyme etc. can easily act on the chain to cleave and/or detect it, and oligonucleotides having a length of about a dozen to several dozens of bases are more preferable for easier detection. The nucleic acid chain may be single-stranded or double-stranded, but for better stability, it is preferable to be double-stranded. The nucleotide sequence of the nucleic acid chain is preferably unique as much as possible for detection by PCR etc.

The nucleic acid chain and the antibody included in the antibody complex are linked via the adaptor moiety. This ensures higher structural stability of the oligonucleotide-complexed antibody and improves the yield of the complex to be obtained, thereby improving the detection sensitivity as well as the detection efficiency and the like.

The construction of the adaptor moiety is not particularly limit as long as it includes the immunoglobulin binding domain of Protein G, Protein A or Protein L.

Therefore, the adaptor moiety may include the entire protein of Protein G, Protein A or Protein L, or a fusion protein of their immunoglobulin binding domain and another peptide. The Protein G, Protein A or Protein L may be a wild-type protein, or a mutant protein having an immunoglobulin binding activity.

For example, the immunoglobulin binding domain is regions at the amino acid positions 303 to 357, 373 to 427 and 443 to 497 in case of Protein G (GenBank accession numbers: cDNA: X06173 and protein: CAA29540); regions at the amino acid positions 39 to 88, 100 to 149, 158 to 207, 216 to 265 and 274 to 323 in case of Protein A (GenBank accession numbers: cDNA: M18264 and protein: AAA26677); and regions at the amino acid positions 115 to 173, 185 to 245, 257 to 317, 329 to 389 and 400 to 462 in case of Protein L (GenBank accession numbers: cDNA: M86697 and protein: AAA25612).

The adaptor moiety may further include a tag if necessary when constructed. The type of the tag is not particularly limited, and may be a GST-tag, an MBP-tag, a myc-tag or a flag-tag, for example. More preferably, the tag is a His-tag, because it can bind to a small nickel molecule, thereby avoiding influence on the chemical cross-linking step.

While the immunoglobulin-binding domain included in the adaptor moiety is directly linked to the antibody, it may be linked to the nucleic acid chain either directly or indirectly. In the cases where it is indirectly linked, the biotin-binding domain of an avidin and the immunoglobulin-binding domain of Protein G, Protein A or Protein L may be linked via a linker compound, or they may form a fusion protein. Meanwhile, the nucleic acid chain may be conjugated with a biotin and the biotin may bind to biotin-binding domain; or both the immunoglobulin-binding domain and the nucleic acid chain may be separately linked to a biotin and these two biotins may be linked via an avidin; or other various embodiments are also possible. As for the linker compound to be used, usable examples include Sulphsuccinimidyl 4-(N-maleimidomethyl) cyclohexane-1-carboxylate (Sulfo-SMCC) and the like.

The avidin usually form a homo-tetramer, and each subunit has one biotin-binding domain, and thus a whole protein has four biotin-binding domains; however, it is sufficient for the biotin binding domain used in the present invention to have only one of such subunits, which may form a tetramer. In order to construct a structure which allows the nucleic acid chain as a label to be exposed into a solution, one molecule of the nucleic acid chain binds preferably to one molecule of Protein G, and therefore it is preferable to use a monomer of an avidin mutant which has only one biotin-binding domain and does not form a tetramer. Although mutants of the avidin which forms a monomer have been extensively studied, there are few successful cases (Qureshi, M. H., and Wong, S. L. (2002). Protein Expr Purif vol.25, p.409-415; Laitinen, O. H.et al., (2003). J Biol Chem vol.278, p.4010-4014) . According to the present invention, however, an avidin mutant that maintains the binding activity to biotin was successfully produced by using a peptide having an amino acid sequence from the position 39 to 183 of streptavidin, as demonstrated in the Examples.

As used herein, the avidin is biotin-binding proteins such as Avidin, streptavidin and NeutrAvidin. For example, the biotin-binding domain is a region at the amino acid position 28 to 146 in the cases of Avidin (RefSeq accession number: cDNA: NM_205320 and protein: NP_990651) and NeutrAvidin, which has the same sequence as avidin but is modified by deglycosylation, and a region at the amino acid position 39 to 156 of streptavidin (GenBank accession numbers: cDNA: X03591, protein: CAA27265).
The adaptor moiety and the antibody are chemically cross-linked. The type of the cross-link is not particularly limited, and examples include cross-links between amino groups, between carboxyl groups and between thiol groups. Although the amino acid residue in the adaptor moiety, which is to be cross-linked to the antibody, is not particularly limited, it is preferably a residue within the immunoglobulin binding domain bound directly to the antibody.

The antibody may be a polyclonal antibody or a monoclonal antibody as long as it can specifically recognize the antigen to be detected. The type of the antibody is not limited, and may be IgG or IgM for example, as long as the antibody can bind with the immunoglobulin binding domain included in the adaptor moiety.

Thus, in an antibody complex including a nucleic acid chain, an antibody and an adaptor moiety linking the nucleic acid chain and the antibody, the sensitivity for detecting the antigen is significantly intensified by cross-linking the adaptor moiety and the antibody to form a cross-linked antibody complex.

The antibody complex may further include a cleavage site capable of releasing the nucleic acid chain. The cleavage site may be provided either in the nucleic acid, in the antibody or in the adaptor moiety. The cleavage site may have different properties depending on the location where it is to be provided: for example, it may be cleaved by a restriction enzyme when it is provided in the nucleic acid; it may be cleaved by a protease when it is provided in the antibody or a protein as the adaptor; and it may be cleaved by photoirradiation or by a reactive oxygen when a cross-linker (divalent cross-linker etc.) is to be provided as the adapter. In terms of simplicity and specificity, it is more preferable to provide a site cleaved by a restriction enzyme in the nucleic acid chain.

In order to make the nucleic acid chain to function as a label, a marker such as a radioactive isotope, a fluorescent dye, an enzyme or the like may be attached to the nucleic acid chain.

### == Method for producing cross-linked antibody complex ==

The cross-linked antibody complex including the nucleic acid chain, the antibody, and the adaptor moiety linking the nucleic acid chain and the antibody may be produced in any method as long as these constituents are to be included.

The complex may be produced by, for example, linking the nucleic acid chain as a label to the adaptor moiety, fixing the adaptor moiety on the antibody to form a an antibody complex, and then cross-linking the adaptor moiety and the antibody using a chemical cross-linker.

In case that the nucleic acid chain is to be linked directly to the adaptor moiety, it may be linked to any position of the adaptor moiety. For example, the link may be formed by modifying a terminal of the nucleic acid chain with an amino group or a thiol group and then chemically linking the modified terminal to a functional group such as amino, carboxyl, thiol etc. in the adaptor moiety using an appropriate cross-linker. In case that the nucleic acid chain is to be linked indirectly to the adaptor moiety, the link may be formed by linking the adaptor moiety to a biotin-binding domain of an avidin, biotinylating the nucleic acid chain and mixing them in a conventional method. Alternatively, the nucleic acid chain may be linked to the adaptor moiety via an avidin by biotinylating both the adaptor moiety and the nucleic acid chain etc. in advance, and mixing them with the avidin in a conventional method. Then, the adaptor moiety-nucleic acid chain complex may be mixed with the antibody in a conventional method to obtain the antibody complex.

In the last step, by treating this antibody complex with a chemical cross-linker, the adaptor moiety and the antibody in the antibody complex are cross-linked. Examples of usable cross-linkers include Dimethyl Pimelimidate (DMP), Dimethyl suberimidate (DMS), Bis[Sulfosuccinimidyl] suberate (BS3) and the like. Among them, DMP is preferably used because of its high efficiency as well as for its high specificity of cross-linking between the antibody complex and the adaptor moiety.

### == Method for detecting antigen with using cross-linked antibody complex ==

The cross-linked antibody complex produced as above is allowed to contact with an antigen to be detected so that an antigen-antibody complex is formed.

The antigen to be detected may be in any type or any form, and it may be a protein, a carbohydrate or a nucleic acid, and may be a purified product or an extract, as long as it can be recognized by the antibody. It may be present in a virus or a cell, in which case the virus or the cell may be used for detection as it is. Therefore, a specimen to be tested may be a component of an organism (e.g., tissue, blood etc.) or its extract, a food product such as meat or vegetable, as well as soil, river water or the like.

The method for producing the antigen-antibody complex is not particularly limit. Immobilization of the antigen on a support is even unnecessary, but for example, a cross-linked antibody complex may be linked to a support by immobilizing the antigen on a support and allowing the cross-linked antibody complex to bind to the antigen. Afterwards, unlinked cross-linked antibody complex may be removed by washing with a buffer to obtain highly pure antigen-antibody complex. As for the support, the bottom surface of a plastic, or beads, may be used. To immobilize the antigen on a support, the antigen may be linked to the support either directly or indirectly. In case of the direct linking, a buffer containing the antigen may be contacted to the support, and in case of the indirect linking, a substance to which the antigen can bind (e.g., an antibody) may be bound with the support in advance, to which a buffer containing the antigen may be contacted. The latter method of indirect linking is more preferable for higher specificity.

In order to detect the cross-linked antibody complex bound with the antigen, the nucleic acid in the cross-linked antibody complex may be detected by applying its container to PCR apparatus etc. as it is. For easier operation of detection, it is more preferable to recover the nucleic acid.

The nucleic acid may be recovered as the entire cross-linked antibody complex. For example, the antigen and the antibody may be separated by a conventional method to recover the cross-linked antibody complex. Alternatively, the nucleic acid alone may be recovered. For example, an acid treatment, an alkaline treatment, a thermal treatment, a protease treatment or the like may be applied to denature or degrade the cross-linked antibody complex.

However, use of such an extreme treatment would make it impossible to achieve the detection by an enzymatic reaction such as HRP reaction, and thus purification of the nucleic acid would become necessary for conducting PCR so that an enzyme such as Taq polymerase can function. Therefore, it is more preferable to provide a cleavage site by which the nucleic acid chain linked to the cross-linked antibody complex can be released by a mild treatment such as a restriction enzyme treatment or a light treatment. Thus, in order to detect the nucleic acid chain, the nucleic acid chain may be released from the antigen-antibody complex at the cleavage site and then recovered. In this recovery step, the nucleic acid chain can be concentrated before conducting the detection, thereby enabling detection of smaller amount of the nucleic acid chain.

The nucleic acid chain thus recovered is detected. The method for detection is not particularly limited, and in the cases where a marker such as a radioisotope, a fluorescent dye or an enzyme is attached to the nucleic acid chain, this marker may be detected. For higher sensitivity of detection, it is more preferable to amplify and detect the nucleic acid chain. The method for amplification may be any of conventional methods usable, such as PCR, LAMP, ICAN and the like. The detection also may be conducted by any of conventional methods such as electrophoresis.

In order to detect multiple antigens simultaneously, antibody complexes, each of which contains an antibody to specifically recognize the respective antigen and a nucleic acid chain corresponding to the antigen, are used so that each of the nucleic acid chains corresponds to its respective antigen. Thus, each of the antigens can be individually detected by detecting its respective nucleic acid chain.

In this case, to obtain the antibody complexes specific for the respective antigens, each of the antibody complexes should be purified after they are prepared separately. And the antibody complexes can be mixed when used.

The specificity of nucleic acid chains for different antigens can be provided by varying their sequences, and/or by varying their lengths. In the latter case, it is possible to utilize a common set of primers for DNA amplification. The specificity can be also provided by labeling the nucleic acid chains with different types of labels (e.g., an alkaline phosphatase and an HRP in case of enzymatic labeling).

### == Kit for detecting antigen ==

To make detection of antigen easier using the cross-linked antibody complex according to the present invention, necessary agents may be assembled as an antigen detection kit.

The kit includes an antibody complex including a nucleic acid chain as a label, an antibody which specifically recognizes an antigen to be detected and an adaptor moiety which links the nucleic acid chain and the antibody, in which the adaptor moiety includes an immunoglobulin binding domain of Protein G, Protein A or Protein L, and the adaptor moiety and the antibody are chemically cross-linked.

Other embodiments of the antibody complex are described above in the "Cross-linked antibody complex" section.

The kit may also include component (s) other than the cross-linked antibody complex, including various buffers and reagents for detection such as primers and enzymes.

### [Examples]

Embodiments of the present invention are hereinafter described more specifically by referring to Examples, which should not be construed as limiting the present invention thereto.

### == Production of fusion protein of Protein G/streptavidin/His-tag ==

First, a fused protein including Protein G / streptavidin / His-tag (hereafter the "fusion protein") was produced as follows. First, the following DNAs were chemically synthesized by a method with leaving phosphate groups unprotected to synthesize: DNA (SEQ ID NO:4, the nucleotide sequence at position 1259 to 1381 in SEQ ID NO:3) which encodes the amino acid sequence (SEQ ID NO:2) at position 228 to 268 in the amino acid sequence set forth in SEQ ID NO: 1 (full-length Protein G, GenBank accession number : M13825) including the region binding to the Fc region of IgG antibody; and DNA (SEQ ID NO:8, the nucleotide sequence at position 164 to 598 in SEQ ID NO:7) which encodes the amino acid sequence (SEQ ID NO:6) at position 39 to 183 in the amino acid sequence set forth in SEQ ID NO:5 (full-length streptavidin, GenBank accession number : X03591) including the region binding to biotin. When necessary, a full-length DNA was prepared by ligating double-stranded DNA fragments individually synthesized. Afterwards, PCR was conducted under the reaction conditions of <35 cycles of 95°C 30 sec - 55°C 30 sec - 72°C 30 sec> using each of the synthesized DNAs as a template and the primers indicated below to amplify respective double-stranded DNAs. The primers were designed so that the DNA fragments to be obtained by PCR should include recognition sites of restriction enzymes at their both ends (nucleotide sequences represented by small letters).

Protein G Primer F:
5'- CATATGCACTTACAAATTAATCCTTAA -3' (SEQ ID NO: 9)
Protein G Primer R:
5'- GAATTCGGATCCTTCACCGTCAACACCGTTG -3' (SEQ ID NO: 10)
Streptavidin Primer F:
5'- GAATTCAAGCTTGCCGGCATCACCGGCACCTG -3' (SEQ ID NO: 11)
Streptavidin Primer R:
5'- CTGCAGCTGCTGAACGGCGTCGAGCG -3' (SEQ ID NO: 12)
The DNA fragments thus obtained were digested with EcoRI, ligated together, and a fused DNA fragment was amplified by PCR using Protein G Primer F and Streptavidin Primer R.

Then, the amplified fused DNA fragment was digested with NdeI, and inserted into an NdeI site of a bacterial expression vector pCR2.1 (Invitrogen Corp.) for synthesizing a fusion protein with a His-tag. The recombinant vector which includes the nucleotide sequence (SEQ ID NO: 14) encoding the fusion protein of Protein G / streptavidin / His-tag (SEQ ID NO: 13) was thus constructed.

The recombinant vector was introduced into E. coli DH5α, the gene expression was induced by IPTG, and the bacteria were solubilized and the fusion protein of Protein G / streptavidin / His-tag was purified using Sepharose beads with immobilized nickel chelate (product name: Ni-NTA agarose, supplier: QIAGEN, product No.: 30210). Fig.1 shows an exemplary result of analysis of the purified protein by SDS-PAGE.

### == Biotinylation of oligonucleotide ==

The Oligonucleotide Chain #1 (SEQ ID NO:15) having a biotinylated 5' terminal PCR was synthesized by PCR using as a template the pcDNA 3 (Invitrogen Corp.) into which DNA having the nucleotide sequence (131 bp) of SEQ ID NO:15 had been inserted under a reaction condition of <35 cycles of 95°C 60 sec - 55°C 60 sec - 72°C 30 sec> with the primers (SEQ ID NO:16 and 17) including biotinylated primers (5-MUSTagBio) indicated below.

#1:

5′-[Biotin]-CACTGCTTACTGGCTTATCGAAATGGAATTCTGCATGCATCTAGAGGGCCCTAT

TCTATAGCATAGTGTCACCTAAATGCTAGGCACCTTCTAGTTGCCAGCCATCTGTTGCACACCAAA

CGTGGCTTGCC-3′ (SEQ ID NO: 15)

Similarly, the Oligonucleotide Chain #7 (SEQ ID NO: 18) having a biotinylated 5' terminal was synthesized using as a template the pcDNA 3 (Invitrogen Corp.) into which DNA having the nucleotide sequence of SEQ ID NO: 18 had been inserted with the same primers (SEQ ID NO: 16, 17).

#7:

5′-[Biotin]-CACTGCTTACTGGCTTATCGAAATGGAATTCTGCATGCATCTAGAGGGCCCTAT

TCTATAGCATAGTGTCACCTAAATGCTAGGCAACCGACAATTGCATGAAGAACTCGCACATTGACG

TCAATAATGACGTATGTTCCCACCACCAAACGTGGCTTGCC-3′ (SEQ ID NO: 18)

### <Sequence of primers for PCR>

5-MUSTag primer Bio-F:
   5'-[Biotin]-CACTGCTTACTGGCTTATCGAAA-3' (SEQ ID NO: 16)
3-MUSTag primer R:
   5'-GGCAAGCCACGTTTGGTG-3' (SEQ ID NO: 17)

### == Production of cross-linked antibody complex ==

### (1) Binding of oligonucleotide and antibody to fusion protein

Into a microcentrifuge tube, 243.4 µl of a binding buffer (0.2 M Borate pH 9.0, 0.5 M NaCl, 0.1 mM EDTA, 0.05% Monocaprate), 6.6 µl of the fusion protein (100 pmol), 40 µl of either of the biotinylated oligonucleotides (#1 for IFN-γ and IL-12; #7 for EGF and IL-15) (100 pmol) were added, and rotated at room temperature for 0.5 hour to bind the streptavidin region of the fusion protein and the biotinylated oligonucleotide. Afterwards, 60 µl each of the antibodies (0.5 mg/ml) indicated below (200 pmol each) was added, and rotated at room temperature for 1 hour to bind the antibody to the Protein G region of the fusion protein.

### <Type of antibodies>

Anti-hEGF antibody: type: goat polyclonal, supplier: R&D, product No. : AF236, concentration: 0.5 mg/mL
Anti-hIFN-γ antibody: type: goat polyclonal, supplier: R&D, product No.: AF-285-NA, concentration: 0.5 mg/mL
Anti-hIL-12 p70 antibody: type: mouse IgGl, supplier: R&D, product No.: MAB611 (clone 24945), concentration: 0.5 mg/mL
Anti-hIL-15 antibody: type: mouse IgGl, supplier: R&D, product No.: MAB247 (clone 34593), concentration: 0.5 mg/mL

### (2) Cross-linking reaction

DMP (Pierce, #21667, MW 259.177) adjusted to 6 mM with a coupling buffer just before use was added to an equal volume (approx. 350 µl) of the reaction solution, mixed well, and then left stand at room temperature for 1 hour. To terminate the cross-linking reaction, 1 M Tris (pH 7.4) was added at the final concentration of 50 mM, left stand at room temperature for 15 minutes, and then filtrated through a 0.45 µm PTFE filter (product name: Millex FH, supplier: Millipore, product No.: SLFHR04NL). Finally, the reaction solution was fractioned by gel filtration chromatography under the conditions described below, and a peak fraction with the highest molecule weight was recovered as the MUSTag solution. The concentration of MUSTag in the solution was determined by comparing with the antibody as a standard, used for the production of the MUSTag, by ELISA.

### <Condition for gel filtration chromatography>

The instrument: product name: SMART system; supplier: (formerly) Pharmacia (presently GE Healthcare) (a discontinued product).
The column: product name: Superdex 200 PC 3.2/30; supplier: GE Healthcare; product No.: 17-1089-01.
The buffer: 10 mM Tris-HCl pH 7.4, 0.5 M NaCl, 0.1 mM EDTA, 0.05% Monocaprate.
The flow rate: 100 µl/min.

### (3) Linking of antigen and MUSTag

The capturing antibodies against EGF, IFN-γ, IL-12 and IL-15 were prepared at the concentration of 2 µg/mL in an immobilization buffer (0.05 M NaHCO₃-Na₂CO₃ Buffer (pH 9.6), 0.02% sodium azide), and 50 µl of each was put in respective wells of a 96 well plate and left at 4°C overnight to immobilize the capturing antibodies onto the plate. After the immobilization, solutions in the wells were discarded, 50 µl of 1% BSA / PBS was added to the wells and left stand at room temperature for 60 minutes to block the plate. After the blocking, 50µl of the antigens diluted at 8 steps of concentrations as described below were added and mixed, and left stand at room temperature for 60 min to bind the antigens to the capturing antibodies immobilized on the plate. After binding to the antibodies, solutions in the wells were discarded, and either the cross-linked MUSTags having been prepared at the concentration of 8 ng/mL using MUSTag-diluting solution (1% BSA / 0.05% Tween 20 / 0.45 M NaCl / 50 mM Na₂HPO₄-NaH₂PO₄ Buffer (pH 7.4)) or, as a control, the MUSTags which were not cross-linked were added by 25 µL each, and left stand at room temperature for 60 min to bind them to the antigens. Then, the wells were washed once with 300 µL of the 1st wash Buffer (0.5 M NaCl / 0.05% Tween 20 / 20 mM Tris-HCl (pH 7.4)), and 3 times with the 2nd wash Buffer (0.05% Tween 20 / PBS 300 µL).

### <Type of capturing antibodies>

Anti-hEGF antibody: type: mouse IgGl; supplier: R&D; product No.: MAB636 (clone 10827); storage concentration: 0.5 mg/mL
Anti-hIFN-γ antibody: type: mouse IgG2A; supplier: R&D; product No.: MAB2852 (clone K3.53); storage concentration: 0.5 mg/mL
Anti-hIL-12 antibody: type: goat polyclonal; supplier: R&D; product No.: AF-219-NA; storage concentration: 0.5 mg/mL
Anti-hIL-15 antibody: type: mouse IgGl; supplier: R&D; product No.: MAB647 (clone 34505); storage concentration: 0.5 mg/mL

### <Preparation of antigen>

For dilution of the antigens, the antigen-diluting solution (1% BSA / PBS) was used.

A stock solution of rhEGF (supplier: R&D, product No.: 236-EG, storage concentration: 200 µg/mL) was diluted by 100, 000-fold to 2000 pg/mL. The diluted antigen solution was further diluted sequentially by 10-fold to prepare 7 steps of 10-fold serial dilutions, and thus the antigen at 8 different concentrations (2000, 200, 20, 2, 0.2, 0.02, 0.002 and 0 [pg/mL]) including a negative control (0 pg/mL, the antigen diluting solution only) were prepared.

A stock solution of rhIFN-γ (supplier: R&D, product No.: 285-IF, storage concentration: 100 µg/mL) was diluted by 5,000-fold to 20000 pg/mL. The diluted antigen solution was further diluted sequentially by 5-fold to prepare 7 steps of 5-fold serial dilutions, and thus the antigen at 8 different concentrations (20000, 4000, 800, 160, 32, 6.4, 1.28 and 0 [pg/mL]) including a negative control (0 pg/mL, the antigen diluting solution only) were prepared.

A stock solution of rhIL-12 (supplier: R&D, product No.: 219-IL, storage concentration: 10 µg/mL) was diluted with 1% BSA / PBS by 250-fold to 40000 pg/mL. The diluted antigen solution was further diluted sequentially by 5-fold to prepare 7 steps of 5-fold serial dilutions, and thus the antigen at 8 different concentrations (40000, 8000, 1600, 320, 64, 12.8, 2.56 and 0 [pg/mL]) including a negative control (0 pg/mL, the antigen diluting solution only) were prepared.

A stock solution of rhIL-15 (supplier: R&D, product No.: 247-IL, storage concentration: 10 µg/mL) was diluted by 2,500-fold to 4000 pg/mL. The diluted antigen solution was further diluted sequentially by 5-fold to prepare 7 steps of 5-fold serial dilutions, and thus the antigen at 8 different concentrations (4000, 800, 160, 32, 6.4, 1.28, 0.256 and 0 [pg/mL]) including a negative control (0 pg/mL, the antigen diluting solution only) were prepared.

### (4) Detection of oligonucleotide chain

Solutions in the wells after the washing were completely discarded, and 30 µL of EcoRI enzyme solution (7.5 unit/mL EcoRI / 50 mM NaCl / 10 mM MgCl2 / 10 mM Tris-HCl (pH 7.4)) was added into the wells (containing EcoRI at 0.225 unit/well) and was reacted at room temperature for 15 min to cleave the oligonucleotide chains of the antibody complexes. The supernatants after the reaction were recovered, and 3 µl of the supernatants was applied to real-time PCR using the following primers and probes (the sequences of the primer were the same between #1 and #7). The PCR was conducted under the conditions of <preheating at 95°C for 15 min; followed by 35 cycles of 95°C, 15 sec - 60°C, 1 min>. The presence of detectable amplified fragments and the changes in the amplified amounts were monitored for each well of the serial dilutions of the antigens by measuring the fluorescent intensity, which varies during the synthesizing reaction of DNA chains in the real-time PCR, at every cycle after the start of the reaction.

### <Sequences of primers and probes for real-time PCR>

Forward primer: 5'-GGGCGGCTGCATCTAGAGGGCCCTATTCTATA-3' (SEQ ID NO: 19)
Reverse primer: 5'-GGCAAGCCACGTTTGGTG-3' (SEQ ID NO: 20)
Probe for #1: 5'-CCTTCTAGTTGCCAGCCATCTGTT-3' (SEQ ID NO: 21)
Probe for #7: 5'-ACCGACAATTGCATGAAGAACTC-3' (SEQ ID NO: 22)

### (5) Normalization and analysis of the result obtained

In the detection method described above, the results of the detection of the antigen-antibody reactions can be represented by the Ct value (the number of cycles required for the amount of DNA amplified by PCR to reach a reference level). The results are shown in Fig.2.

Based on the results of the detection, a differential (ΔCt) was calculated by subtracting the Ct value at the antigen concentration from the average of Ct values of blanks to normalize calibration curves. The results are shown in Fig.3.

A concentration of the antigen where the ΔCt has reached 3.3 times as much as the measured value of the blank in the measurement system was taken as the detection limit at the lower side of concentration, and (the antigen concentration at the lower-side detection limit when the cross-linked MUSTag was used) / (the antigen concentration at the lower-side detection limit when a MUSTag without cross-link was used) was taken as the detection sensitivity. It should be noted that the value of sensitivity when the MUSTag without cross-link was used was regarded as 1. Similarly, the quantification limit on the upper side of concentration was obtained as a concentration where the coefficient of variation (C.V.) of the converted values became 20% in converting the ΔCt at the antigen concentration into concentration using the calibration curve obtained.

The detection limits at the lower side of concentration as well as the detection sensitivities and the quantification limits at the upper side of concentration obtained by using the cytokines are shown in Table 1.

**[Table 1]**

| | | Detection limit | | Quantification limit (upper) | |
|---|---|---|---|---|---|
| Cytokine | Cross-linker | Ag conc. (pg/mL) | Efficiency | Ag conc. (pg/mL) | Efficiency |
| EGF #7 | No crosslink | 0.1981527 | 1 | 2290.5922 | 1 |
| (capture: mono → MUSTag: poly) | Crosslinked | 0.049351 | 4.0151734 | 3843.8635 | 1.6781091 |
| IFN-γ #1 | No crosslink | 1 1.38072 | 1 | 1 0899.282 | 1 |
| (capture: mono → MUSTag: poly) | Crosslinked | 3.521915 | 3.231401 1 | 20843.85 | 1.91 28646 |
| IL-12#1 | No crosslink | 983.3599 | 1 | 1 7463.556 | 1 |
| (capture: poly → MUSTag: mono) | Crosslinked | 32.32467 | 30.421 344 | 41759.316 | 2.391 2264 |
| IL-15#7 (capture: mono → MUSTag: mono) | No crosslink | 1 10.7488 | 1 | 1 256.4623 | 1 |
| | 2 mM DMP | 1.31649 | 84.1 243 | 1 206.8069 | 0.96048 |
| | 2 mM DMS | 0.91 53934 | 1 20.98492 | 1841.1857 | 1.4653728 |
| | 10 mM BS³ | 8.38939 | 13.201055 | 1 434.9854 | 1.1420839 |
| | 5 mM BS³ | 7.113186 | 1 5.569507 | 1 560.0045 | 1.2415848 |

### (6) Comparison of sensitivities with and without cross-link

The cross-linking reaction brought improvements in sensitivity of 3 to 5 times in the cases using the MUSTag made from the goat polyclonal antibody (EGF, IFN-γ) , and 20 to 100 times in the cases using the MUSTag made from the mouse monoclonal antibody (IL-12, IL-15) . In addition to the maximum sensitivities at lower concentrations, the quantification limits at the upper side of concentration were also improved, thereby expanding the entire measurable range.

With regard to the signal intensities for the ΔCt values corresponding to the same antigen concentration, increases in the ΔCt values of 2 times at maximum with the goat polyclonal antibody, and 5 times at maximum with the mouse monoclonal antibody, could be observed. Accordingly, the use of the cross-linked MUSTag improves the S/N ratio of measurements, thereby enabling more accurate quantification.

### (7) Comparison of sensitivity between the cross-linkers

By using DMS (PIERCE, #20700, MW: 273.2, final concentration: 2 mM) or BS³ (PIERCE, #21580, MW: 572.43, final concentration: 5 mM or 10 mM) in place of DMP as the cross-linker, experiments of detection were conducted for the antigen IL-15, and the results are shown in Fig.4. The MUSTag cross-linked by DMS in this experiment showed a similar reactivity to the MUSTag cross-linked by DMP. Also in the case of the cross-linking by BS3, obvious improvements in the sensitivity and range width were observed in comparison to the control.

Accordingly, the cross-linking reaction can improve reactivity of the MUSTag, regardless of the types of the amine reactive cross-linkers.

### [Industrial Applicability]

According to the present invention, the detection sensitivity of MUSTag can be improved.

## Claims

1. An antibody complex for detecting an antigen, comprising a nucleic acid chain as a label, an antibody to specifically recognize the antigen, and a adaptor moiety linking the nucleic acid chain and the antibody, wherein
the adaptor moiety comprises an immunoglobulin binding domain of Protein G, Protein A or Protein L, and
the adaptor moiety and the antibody are chemically cross-linked.

2. The antibody complex according to Claim 1, comprising a cleavage site capable of releasing the nucleic acid chain.

3. The antibody complex according to Claim 2, wherein the cleavage site is cleaved by a restriction enzyme, by photoirradiation or by reactive oxygen.

4. The antibody complex according to any one of Claims 1 to 3, wherein the adaptor moiety comprises a fusion protein comprising a biotin binding domain of an avidin and the immunoglobulin binding domain of Protein G, Protein A or Protein L, and the nucleic acid chain is a biotin-conjugated nucleic acid.

5. The antibody complex according to any one of Claims 1 to 4, wherein the antibody is a monoclonal antibody.

6. A method for detecting an antigen, comprising the steps of:
allowing the antibody complex according to any one of Claims 1 to 5 to contact with the antigen, thereby forming an antigen-antibody complex comprising the antigen and the antibody complex, and
detecting the oligonucleotide chain.

7. The method according to Claim 6, wherein
the antigen-antibody complex comprises the antibody complex according to Claim 2 or 3, and
the step of detecting the oligonucleotide chain comprises the step of releasing the oligonucleotide chain at the cleavage site and recovering the oligonucleotide chain.

8. The method according to Claim 6 or 7, wherein the step of detecting the oligonucleotide chain comprises the steps of:
amplifying the oligonucleotide chain, and
detecting the amplified oligonucleotide chain.

9. A kit for detecting an antigen, comprising
a nucleic acid chain as a label, an antibody to specifically recognize the antigen, and an antibody complex comprising an adaptor moiety linking the nucleic acid chain and the antibody, wherein
the adaptor moiety comprises an immunoglobulin binding domain of Protein G, Protein A or Protein L, and
the adaptor moiety and the antibody are chemically cross-linked.

10. The kit according to Claim 9, wherein the antibody complex comprises a cleavage site capable of releasing the nucleic acid chain.

11. The kit according to Claim 10, wherein the cleavage site is cleaved by a restriction enzyme, by photoirradiation or by reactive oxygen.

12. The kit according to any one of Claims 9 to 11, wherein the adaptor moiety comprises a fusion protein comprising a biotin-binding domain of an avidin and the immunoglobulin-binding domain of Protein G, Protein A or Protein L, and the nucleic acid chain is a biotin-conjugated nucleic acid.

13. The kit according to any one of Claims 9 to 12, wherein the antibody is a monoclonal antibody.


SEQUENCE LISTING

<110>  Tokyo Metropolitan Organization for Medical Research
       Synthera Technologies

<120>  Antibody complex, method for detecting antibody , and method for
       making antibody complex

<130>  PCT1568

<150>  JP 2008/171512
<151>  2008-06-30

<160>  22

<170>  PatentIn version 3.5

<210>  1
<211>  448
<212>  PRT
<213>  Streptococcus sp.

<400>  1

Met Glu Lys Glu Lys Lys Val Lys Tyr Phe Leu Arg Lys Ser Ala Phe
1               5                   10                  15

Gly Leu Ala Ser Val Ser Ala Ala Phe Leu Val Gly Ser Thr Val Phe
            20                  25                  30

Ala Val Asp Ser Pro Ile Glu Asp Thr Pro Ile Ile Arg Asn Gly Gly
        35                  40                  45

Glu Leu Thr Asn Leu Leu Gly Asn Ser Glu Thr Thr Leu Ala Leu Arg
    50                  55                  60

Asn Glu Glu Ser Ala Thr Ala Asp Leu Thr Ala Ala Ala Val Ala Asp
65                  70                  75                  80

Thr Val Ala Ala Ala Ala Ala Glu Asn Ala Gly Ala Ala Ala Trp Glu
                85                  90                  95

Ala Ala Ala Ala Ala Asp Ala Leu Ala Lys Ala Lys Ala Asp Ala Leu
            100                 105                 110

Lys Glu Phe Asn Lys Tyr Gly Val Ser Asp Tyr Tyr Lys Asn Leu Ile
        115                 120                 125

Asn Asn Ala Lys Thr Val Glu Gly Ile Lys Asp Leu Gln Ala Gln Val
    130                 135                 140

Val Glu Ser Ala Lys Lys Ala Arg Ile Ser Glu Ala Thr Asp Gly Leu
145                 150                 155                 160

Ser Asp Phe Leu Lys Ser Gln Thr Pro Ala Glu Asp Thr Val Lys Ser
                165                 170                 175

Ile Glu Leu Ala Glu Ala Lys Val Leu Ala Asn Arg Glu Leu Asp Lys
            180                 185                 190

Tyr Gly Val Ser Asp Tyr His Lys Asn Leu Ile Asn Asn Ala Lys Thr
        195                 200                 205

Val Glu Gly Val Lys Glu Leu Ile Asp Glu Ile Leu Ala Ala Leu Pro
    210                 215                 220

Lys Thr Asp Thr Tyr Lys Leu Ile Leu Asn Gly Lys Thr Leu Lys Gly
225                 230                 235                 240

Glu Thr Thr Thr Glu Ala Val Asp Ala Ala Thr Ala Glu Lys Val Phe
                245                 250                 255

Lys Gln Tyr Ala Asn Asp Asn Gly Val Asp Gly Glu Trp Thr Tyr Asp
            260                 265                 270

Asp Ala Thr Lys Thr Phe Thr Val Thr Glu Lys Pro Glu Val Ile Asp
        275                 280                 285

Ala Ser Glu Leu Thr Pro Ala Val Thr Thr Tyr Lys Leu Val Ile Asn
    290                 295                 300

Gly Lys Thr Leu Lys Gly Glu Thr Thr Thr Lys Ala Val Asp Ala Glu
305                 310                 315                 320

Thr Ala Glu Lys Ala Phe Lys Gln Tyr Ala Asn Asp Asn Gly Val Asp
                325                 330                 335

Gly Val Trp Thr Tyr Asp Asp Ala Thr Lys Thr Phe Thr Val Thr Glu
            340                 345                 350

Met Val Thr Glu Val Pro Gly Asp Ala Pro Thr Glu Pro Glu Lys Pro
        355                 360                 365

Glu Ala Ser Ile Pro Leu Val Pro Leu Thr Pro Ala Thr Pro Ile Ala
    370                 375                 380

Lys Asp Asp Ala Lys Lys Asp Asp Thr Lys Lys Glu Asp Ala Lys Lys
385                 390                 395                 400

Pro Glu Ala Lys Lys Asp Asp Ala Lys Lys Ala Glu Thr Leu Pro Thr
                405                 410                 415

Thr Gly Glu Gly Ser Asn Pro Phe Phe Thr Ala Ala Ala Leu Ala Val
            420                 425                 430

Met Ala Gly Ala Gly Ala Leu Ala Val Ala Ser Lys Arg Lys Glu Asp
        435                 440                 445

<210>  2
<211>  41
<212>  PRT
<213>  Streptococcus sp.

<400>  2

Thr Tyr Lys Leu Ile Leu Asn Gly Lys Thr Leu Lys Gly Glu Thr Thr
1               5                   10                  15

Thr Glu Ala Val Asp Ala Ala Thr Ala Glu Lys Val Phe Lys Gln Tyr
            20                  25                  30

Ala Asn Asp Asn Gly Val Asp Gly Glu
        35                  40

<210>  3
<211>  1950
<212>  DNA
<213>  Streptococcus sp.

<400>  3
aagctttggt ggagaaattg gctggcgaat ccagcttcac cggtgtttca ccagtagatg      60

ctttctgtgg tcttattgac acgcacttgt ggcgagagta ctaacagtca cagcgacgtt     120

aactttattt tccttatgag aggttaagaa aaaacgttat taaatagcag aaaagaatat     180

tatgactgac gttaggagtt ttctcctaac gtttttttta gtacaaaaag agaattctct     240

attataaata aaataaatag tactatagat agaaaatctc atttttaaaa agtcttgttt     300

tcttaaagaa gaaaataatt gttgaaaaat tatagaaaat catttttata ctaatgaaat     360

agacataagg ctaaattggt gaggtgatga taggagattt atttgtaagg attccttaat     420

tttattaatt caacaaaaat tgatagaaaa attaaatgga atccttgatt taattttatt     480

aagttgtata ataaaaagtg aaattattaa atcgtagttt caaatttgtc ggctttttaa     540

tatgtgctgg catattaaaa ttaaaaaagg agaaaaaatg gaaaaagaaa aaaaggtaaa     600

atacttttta cgtaaatcag cttttgggtt agcatccgta tcagctgcat ttttagtggg     660

atcaacggta ttcgctgttg attcaccaat cgaagatacc ccaattattc gtaatggtgg     720

tgaattaact aatcttctgg ggaattcaga gacaacactg gctttgcgta atgaagagag     780

tgctacagct gatttgacag cagcagcggt agccgatact gtggcagcag cggcagctga     840

aaatgctggg gcagcagctt gggaagcagc ggcagcagca gatgctctag caaaagccaa     900

agcagatgcc cttaaagaat tcaacaaata tggagtaagt gactattaca agaatctaat     960

caacaatgcc aaaactgttg aaggcataaa agaccttcaa gcacaagttg ttgaatcagc    1020

gaagaaagcg cgtatttcag aagcaacaga tggcttatct gatttcttga aatcgcaaac    1080

acctgctgaa gatactgtta aatcaattga attagctgaa gctaaagtct tagctaacag    1140

agaacttgac aaatatggag taagtgacta tcacaagaac ctaatcaaca atgccaaaac    1200

tgttgaaggt gtaaaagaac tgatagatga aattttagct gcattaccta agactgacac    1260

ttacaaatta atccttaatg gtaaaacatt gaaaggcgaa acaactactg aagctgttga    1320

tgctgctact gcagaaaaag tcttcaaaca atacgctaac gacaacggtg ttgacggtga    1380

atggacttac gacgatgcga ctaagacctt tacagttact gaaaaaccag aagtgatcga    1440

tgcgtctgaa ttaacaccag ccgtgacaac ttacaaactt gttattaatg gtaaaacatt    1500

gaaaggcgaa acaactacta aagcagtaga cgcagaaact gcagaaaaag ccttcaaaca 1560

atacgctaac gacaacggtg ttgatggtgt ttggacttat gatgatgcga ctaagacctt 1620

tacggtaact gaaatggtta cagaggttcc tggtgatgca ccaactgaac cagaaaaacc 1680

agaagcaagt atccctcttg ttccgttaac tcctgcaact ccaattgcta aagatgacgc 1740

taagaaagac gatactaaga aagaagatgc taaaaaacca gaagctaaga aagatgacgc 1800

taagaaagct gaaactcttc ctacaactgg tgaaggaagc aacccattct tcacagcagc 1860

tgcgcttgca gtaatggctg gtgcgggtgc tttggcggtc gcttcaaaac gtaaagaaga 1920

ctaattgtca ttatttttga caaaaagctt 1950

<210> 4
<211> 123
<212> DNA
<213> Streptococcus sp.

<400> 4
acttacaaat taatccttaa tggtaaaaca ttgaaaggcg aaacaactac tgaagctgtt 60

gatgctgcta ctgcagaaaa agtcttcaaa caatacgcta acgacaacgg tgttgacggt 120

gaa 123

<210> 5
<211> 183
<212> PRT
<213> Streptomyces avidinii

<400> 5

Met Arg Lys Ile Val Val Ala Ala Ile Ala Val Ser Leu Thr Thr Val
1               5                   10                  15

Ser Ile Thr Ala Ser Ala Ser Ala Asp Pro Ser Lys Asp Ser Lys Ala
            20                  25                  30

Gln Val Ser Ala Ala Glu Ala Gly Ile Thr Gly Thr Trp Tyr Asn Gln
        35                  40                  45

Leu Gly Ser Thr Phe Ile Val Thr Ala Gly Ala Asp Gly Ala Leu Thr
    50                  55                  60

Gly Thr Tyr Glu Ser Ala Val Gly Asn Ala Glu Ser Arg Tyr Val Leu
65                  70                  75                  80

Thr Gly Arg Tyr Asp Ser Ala Pro Ala Thr Asp Gly Ser Gly Thr Ala
                85                  90                  95

Leu Gly Trp Thr Val Ala Trp Lys Asn Asn Tyr Arg Asn Ala His Ser
            100                 105                 110

Ala Thr Thr Trp Ser Gly Gln Tyr Val Gly Gly Ala Glu Ala Arg Ile
        115                 120                 125

Asn Thr Gln Trp Leu Leu Thr Ser Gly Thr Thr Glu Ala Asn Ala Trp
    130                 135                 140

Lys Ser Thr Leu Val Gly His Asp Thr Phe Thr Lys Val Lys Pro Ser
145                 150                 155                 160

Ala Ala Ser Ile Asp Ala Ala Lys Lys Ala Gly Val Asn Asn Gly Asn
                165                 170                 175

Pro Leu Asp Ala Val Gln Gln
            180

<210>  6
<211>  145
<212>  PRT
<213>  Streptomyces avidinii

<400>  6

Ala Gly Ile Thr Gly Thr Trp Tyr Asn Gln Leu Gly Ser Thr Phe Ile
1               5                   10                  15

Val Thr Ala Gly Ala Asp Gly Ala Leu Thr Gly Thr Tyr Glu Ser Ala
            20                  25                  30

Val Gly Asn Ala Glu Ser Arg Tyr Val Leu Thr Gly Arg Tyr Asp Ser
        35                  40                  45

Ala Pro Ala Thr Asp Gly Ser Gly Thr Ala Leu Gly Trp Thr Val Ala
    50                  55                  60

Trp Lys Asn Asn Tyr Arg Asn Ala His Ser Ala Thr Thr Trp Ser Gly
65                  70                  75                  80

Gln Tyr Val Gly Gly Ala Glu Ala Arg Ile Asn Thr Gln Trp Leu Leu
                85                  90                  95

Thr Ser Gly Thr Thr Glu Ala Asn Ala Trp Lys Ser Thr Leu Val Gly
            100                 105                 110

His Asp Thr Phe Thr Lys Val Lys Pro Ser Ala Ala Ser Ile Asp Ala
        115                 120                 125

Ala Lys Lys Ala Gly Val Asn Asn Gly Asn Pro Leu Asp Ala Val Gln
    130                 135                 140

Gln
145

<210>  7
<211>  638
<212>  DNA
<213>  Streptomyces avidinii

<400>  7
ccctccgtcc ccgccgggca acaactaggg agtatttttc gtgtctcaca tgcgcaagat       60

cgtcgttgca gccatcgccg tttccctgac cacggtctcg attacggcca gcgcttcggc 120

agacccctcc aaggactcga aggcccaggt ctcggccgcc gaggccggca tcaccggcac 180

ctggtacaac cagctcggct cgaccttcat cgtgaccgcg ggcgccgacg gcgccctgac 240

cggaacctac gagtcggccg tcggcaacgc cgagagccgc tacgtcctga ccggtcgtta 300

cgacagcgcc ccggccaccg acggcagcgg caccgccctc ggttggacgg tggcctggaa 360

gaataactac cgcaacgccc actccgcgac cacgtggagc ggccagtacg tcggcggcgc 420

cgaggcgagg atcaacaccc agtggctgct gacctccggc accaccgagg ccaacgcctg 480

gaagtccacg ctggtcggcc acgacacctt caccaaggtg aagccgtccg ccgcctccat 540

cgacgcggcg aagaaggccg gcgtcaacaa cggcaacccg ctcgacgccg ttcagcagta 600

gtcgcgtccc ggcaccggcg ggtgccggga cctcggcc 638

<210> 8
<211> 435
<212> DNA
<213> Streptomyces avidinii

<400> 8
gccggcatca ccggcacctg gtacaaccag ctcggctcga ccttcatcgt gaccgcgggc 60

gccgacggcg ccctgaccgg aacctacgag tcggccgtcg gcaacgccga gagccgctac 120

gtcctgaccg gtcgttacga cagcgccccg gccaccgacg gcagcggcac cgccctcggt 180

tggacggtgg cctggaagaa taactaccgc aacgcccact ccgcgaccac gtggagcggc 240

cagtacgtcg gcggcgccga ggcgaggatc aacacccagt ggctgctgac ctccggcacc 300

accgaggcca acgcctggaa gtccacgctg gtcggccacg acaccttcac caaggtgaag 360

ccgtccgccg cctccatcga cgcggcgaag aaggccggcg tcaacaacgg caacccgctc 420

gacgccgttc agcag 435

<210> 9
<211> 27
<212> DNA
<213> Artificial Sequence

<220>
<223> PCR primer

<400> 9
catatgcact tacaaattaa tccttaa 27

<210> 10
<211> 31
<212> DNA
<213> Artificial Sequence

<220>
<223> PCR primer

<400> 10
gaattcggat ccttcaccgt caacaccgtt g 31

<210> 11

<211>  32
<212>  DNA
<213>  Artificial Sequence

<220>
<223>  PCR primer

<400>  11
gaattcaagc ttgccggcat caccggcacc tg                                     32


<210>  12
<211>  26
<212>  DNA
<213>  Artificial Sequence

<220>
<223>  PCR primer

<400>  12
ctgcagctgc tgaacggcgt cgagcg                                            26


<210>  13
<211>  301
<212>  PRT
<213>  Artificial Sequence

<220>
<223>  fusion protein

<400>  13

Met Thr Met Ile Thr Pro Ser Leu Val Pro Ser Ser Asp Pro Leu Val
1               5                   10                  15

Thr Ala Ala Ser Val Leu Glu Phe Gly Phe Ile Cys Thr Tyr Lys Leu
            20                  25                  30

Ile Leu Asn Gly Lys Thr Leu Lys Gly Glu Thr Thr Thr Glu Ala Val
        35                  40                  45

Asp Ala Ala Thr Ala Glu Lys Val Phe Lys Gln Tyr Ala Asn Asp Asn
    50                  55                  60

Gly Val Asp Gly Glu Gly Ser Glu Phe Lys Leu Ala Gly Ile Thr Gly
65                  70                  75                  80

Thr Trp Tyr Asn Gln Leu Gly Ser Thr Phe Ile Val Thr Ala Gly Ala
                85                  90                  95

Asp Gly Ala Leu Thr Gly Thr Tyr Glu Ser Ala Val Gly Asn Ala Glu
            100                 105                 110

Ser Arg Tyr Val Leu Thr Gly Arg Tyr Asp Ser Ala Pro Ala Thr Asp
        115                 120                 125

Gly Ser Gly Thr Ala Leu Gly Trp Thr Val Ala Trp Lys Asn Asn Tyr
    130                 135                 140

Arg Asn Ala His Ser Ala Thr Thr Trp Ser Gly Gln Tyr Val Gly Gly

145                 150                 155                 160

Ala Glu Ala Arg Ile Asn Thr Gln Trp Leu Leu Thr Ser Gly Thr Thr
                165                 170                 175

Glu Ala Asn Ala Trp Lys Ser Thr Leu Val Gly His Asp Thr Phe Thr
            180                 185                 190

Lys Val Lys Pro Ser Ala Ala Ser Ile Asp Ala Ala Lys Lys Ala Gly
        195                 200                 205

Val Asn Asn Gly Asn Pro Leu Asp Ala Val Gln Gln Leu Gln Lys Pro
    210                 215                 220

Asn Ser Ala Asp Ile His His Thr Gly Gly Arg Ser Ser Met His Leu
225                 230                 235                 240

Glu Gly Pro Ile Arg Pro Ile Val Ser Arg Ile Thr Ile His Trp Pro
                245                 250                 255

Ser Phe Tyr Asn Val Val Thr Gly Lys Thr Leu Ala Leu Pro Asn Leu
            260                 265                 270

Ile Ala Leu Gln His Ile Pro Leu Ser Pro Ala Gly Val Ile Ala Lys
        275                 280                 285

Arg Pro Ala Pro Ile Ala Leu Pro Asn Ser Cys Ala Ala
    290                 295                 300

<210>  14
<211>  906
<212>  DNA
<213>  Artificial Sequence

<220>
<223>  DNA encoding the fusin protein

<400>  14
atgaccatga ttacgccaag cttggtaccg agctcggatc cactagtaac ggccgccagt      60

gtgctggaat tcggcttcat atgcacttac aaattaatcc ttaatggtaa aacattgaaa     120

ggcgaaacaa ctactgaagc tgttgatgct gctactgcag aaaaagtctt caaacaatac     180

gctaacgaca acggtgttga cggtgaagga tccgaattca agcttgccgg catcaccggc     240

acctggtaca accagctcgg ctcgaccttc atcgtgaccg cgggcgccga cggcgccctg     300

accggaacct acgagtcggc cgtcggcaac gccgagagcc gctacgtcct gaccggtcgt     360

tacgacagcg ccccggccac cgacggcagc ggcaccgccc tcggttggac ggtggcctgg     420

aagaataact accgcaacgc ccactccgcg accacgtgga gcggccagta cgtcggcggc     480

gccgaggcga ggatcaacac ccagtggctg ctgacctccg gcaccaccga ggccaacgcc     540

tggaagtcca cgctggtcgg ccacgacacc ttcaccaagg tgaagccgtc cgccgcctcc     600

atcgacgcgg cgaagaaggc cggcgtcaac aacggcaacc cgctcgacgc cgttcagcag     660

ctgcagaagc cgaattctgc agatatccat cacactggcg gccgctcgag catgcatcta 720

gagggcccaa ttcgccctat agtgagtcgt attacaattc actggccgtc gttttacaac 780

gtcgtgactg ggaaaaccct ggcgttaccc aacttaatcg ccttgcagca catccccctt 840

tcgccagctg gcgtaatagc gaagaggccc gcaccgatcg cccttcccaa cagttgcgca 900

gcctga 906

<210> 15
<211> 131
<212> DNA
<213> Artificial Sequence

<220>
<223> Oligo #1

<400> 15
cactgcttac tggcttatcg aaatggaatt ctgcatgcat ctagagggcc ctattctata 60

gcatagtgtc acctaaatgc taggcacctt ctagttgcca gccatctgtt gcacaccaaa 120

cgtggcttgc c 131

<210> 16
<211> 23
<212> DNA
<213> Artificial Sequence

<220>
<223> PCR primer

<400> 16
cactgcttac tggcttatcg aaa 23

<210> 17
<211> 18
<212> DNA
<213> Artificial Sequence

<220>
<223> PCR primer

<400> 17
ggcaagccac gtttggtg 18

<210> 18
<211> 161
<212> DNA
<213> Artificial Sequence

<220>
<223> Oligo #7

<400> 18
cactgcttac tggcttatcg aaatggaatt ctgcatgcat ctagagggcc ctattctata 60

gcatagtgtc acctaaatgc taggcaaccg acaattgcat gaagaactcg cacattgacg 120

tcaataatga cgtatgttcc caccaccaaa cgtggcttgc c 161

<210> 19
<211> 32
<212> DNA

<213>  Artificial Sequence

<220>
<223>  PCR primer

<400>  19
gggcggctgc atctagaggg ccctattcta ta                                    32


<210>  20
<211>  18
<212>  DNA
<213>  Artificial Sequence

<220>
<223>  PCR primer

<400>  20
ggcaagccac gtttggtg                                                    18


<210>  21
<211>  24
<212>  DNA
<213>  Artificial Sequence

<220>
<223>  PCR probe

<400>  21
ccttctagtt gccagccatc tgtt                                             24


<210>  22
<211>  23
<212>  DNA
<213>  Artificial Sequence

<220>
<223>  PCR probe

<400>  22
accgacaatt gcatgaagaa ctc                                              23